# EUROPEAN PATENT APPLICATION

(11) **EP 4 840 971 A1**
(43) Date of publication of application: **18.11.2026**
(21) Application number: 25177179.6
(22) Date of filing: 19.05.2025
(51) Int. Cl.: C12Q 1/6886

(54) **A SET OF OLIGONUCLEOTIDE PROBES AND A METHOD FOR DETERMINING IN VITRO PREDICTION OF THE OCCURRENCE OF RADIATION-RELATED CONDITIONS**

(30) Priority: 16.05.2025 PL 45205625
(71) Applicant: Gdanski Uniwersytet Medyczny, 80-210 Gdansk (PL)
(72) Inventor: Perdyan, Adrian, 02-760 Warszawa (PL); awrynowicz, Urszula, 80-034 Gdansk (PL); Piotrowski, Arkadiusz, 83-031 Ró yny (PL); Mieczkowski, Jakub, 80-438 Gda sk (PL)
(74) Representative: JD&P Patent Attorneys Joanna Dargiewicz & Partners

(57) **Abstract**

The subject of the invention is a set of differentially methylated oligonucleotide probes (DMPs) for predicting the occurrence of radiation-related disease states, characterized in that the set comprises eighteen probes, having the sequences identified as SEQ ID NO: 1-18. The subject of the invention also includes a method for *in vitro* prediction of radiation-related disease states by analyzing epigenetic changes in the genome of an individual.

Sequence List:

<110> GDAŃSKI UNIWERSYTET MEDYCZNY

<120> A set of oligonucleotide probes and a method for in vitro prediction of radiation-
related disease states

<130> 66031

<160> 18

<170> BiSSAP 1.3.6

<210> 1

<211> 30

<212> DNA

<213> Artificial sequence

<220>

<223> oligonucleotide probe

<400> 1

tttttttaaa agttagaatt taagttttaa                                30

<210> 2

<211> 25

<212> DNA

<213> Artificial sequence

<220>

<223> oligonucleotide probe

<400> 2

ataacacaat aaaaccccaa caaac                                25

<210> 3

<211> 26

<212> DNA

<213> Artificial sequence

<220>
<223> oligonucleotide probe
<400> 3
attagggttt atgtttttta tgaagg 26

<210> 4
<211> 25
<212> DNA
<213> Artificial sequence

<220>
<223> oligonucleotide probe
<400> 4
caaaaaacct tacaatatca cttcc 25

<210> 5
<211> 25
<212> DNA
<213> Artificial sequence

<220>
<223> oligonucleotide probe
<400> 5
aaggagttgg aagataattt ttttt 25

<210> 6
<211> 25
<212> DNA
<213> Artificial sequence

<220>
<223> oligonucleotide probe

<400> 6
tcaaaaaaca caaaactcaa taaac 25

<210> 7
<211> 30
<212> DNA
<213> Artificial sequence

<220>
<223> oligonucleotide probe
<400> 7
tgtataataa gtattgagag taattagagg 30

<210> 8
<211> 25
<212> DNA
<213> Artificial sequence

<220>
<223> oligonucleotide probe
<400> 8
atccaaatca aaaaacacta ttttt 25

<210> 9
<211> 23
<212> DNA
<213> Artificial sequence

<220>
<223> oligonucleotide probe
<400> 9
ttttagagag ttttaggagg agg 23

<210> 10

<211> 26

<212> DNA

<213> Artificial sequence

<220>

<223> oligonucleotide probe

<400> 10

aacaattatt ttaaaaacaa aaaaaa 26

<210> 11

<211> 27

<212> DNA

<213> Artificial sequence

<220>

<223> oligonucleotide probe

<400> 11

ttagagaagg ttttgtgatg gataatt 27

<210> 12

<211> 25

<212> DNA

<213> Artificial sequence

<220>

<223> oligonucleotide probe

<400> 12

taaaaacaaa ccccataaaa atcac 25

<210> 13

<211> 26

<212> DNA
<213> Artificial sequence

<220>
<223> oligonucleotide probe
<400> 13
tggagttaaa tttatttgtt tttttt 26

<210> 14
<211> 25
<212> DNA
<213> Artificial sequence

<220>
<223> oligonucleotide probe
<400> 14
caaaaatcat aatttccacc ctaaa 25

<210> 15
<211> 25
<212> DNA
<213> Artificial sequence

<220>
<223> oligonucleotide probe
<400> 15
tagggttgaa atagaggttt ttttt 25

<210> 16
<211> 24
<212> DNA
<213> Artificial sequence

<220>
<223> oligonucleotide probe
<400> 16
acacaaaatc taaccctaca ctcc 24

<210> 17
<211> 25
<212> DNA
<213> Artificial sequence

<220>
<223> oligonucleotide probe
<400> 17
tattagtttt ttgttaattg gagag 25

<210> 18
<211> 25
<212> DNA
<213> Artificial sequence

<220>
<223> oligonucleotide probe
<400> 18
accaaaacca cctaataacc ctaac 25

## Description

The subject of the invention is a set of differentially methylated oligonucleotide probes (DMPs) for predicting the occurrence of radiation-related disease states, and a method for *in vitro* prediction of such disease states by analyzing epigenetic changes in the genome of an individual, wherein the DNA methylation profile enables identification of exposure to a high dose of 56Fe heavy ion irradiation.

State-of-the-art studies by Kennedy et al. [1] have demonstrated that exposure to heavy ion radiation induces persistent changes in the epigenome, which may be relevant to the development of human lung carcinoma. Specific alterations in DNA methylation, particularly at CpG sites, are capable of distinguishing healthy tissue from cancerous tissue, such as lung adenocarcinoma or squamous cell lung carcinoma. Moreover, these methylation changes are stable over time, indicating their potential relevance to carcinogenic processes associated with exposure to cosmic radiation. Studies by Nwanaji-Enwerem et al. [2] have further demonstrated that heavy ion radiation affects epigenetic clocks in human bronchial epithelial cells. Such changes may accelerate cellular aging processes and contribute to an increased risk of age-related diseases, including cancer.

Publication [3] demonstrates three-dimensional spatial patterns of DNA methylation induced by high linear energy transfer (LET) radiation (56Fe, 28Si) and low-LET radiation (X-rays), as well as the influence of chromosome positioning and epigenetic architecture across different radial layers of the cell nucleus. It was shown that 56Fe particles induce a sustained increase in DNA methylation, whereas 28Si and X-ray radiation lead to a transient decrease in DNA methylation.

The publication by Wang et al. [4] demonstrates that changes in enhancer activity can fundamentally alter the regulation of gene expression, even in the presence of an identical genetic background. The MACC method, described by Mieczkowski et al. [5], enables precise differentiation between nucleosome accessibility and occupancy, revealing new principles of transcriptional regulation, which were further confirmed in the context of cellular stress responses in studies by Mueller et al. [6]. Moreover, the dynamics of histone turnover indicate that active enhancers are characterized by exceptionally high variability and histone exchange [7]. Three-dimensional maps of DNA-DNA interactions, integrated with data on chromatin accessibility, DNA methylation, and gene expression levels, allow for the identification of active regulatory elements and their spatial interactions, thereby revealing epigenetic mechanisms governing transcription [8]. The transcriptional response to factors altering DNA-DNA interactions depends on the activity of signaling pathways influenced by the underlying genetic background [9]. The publication by Tiwari et al. [10] shows that cellular senescence results in epigenetic closure of chromatin accessibility at regeneration-associated loci, which can be reversed through pharmacological modulation. The publication by Leszczynska et al. [11] describes a mechanism whereby pharmacological inhibition of histone deacetylation leads to the selective degradation of specific proteins via pathways dependent on non-canonical histone variants and lysosomal degradation, enabling therapeutic modulation of altered chromatin architecture in cancer.

The technical problem in the current state of the art is the lack of precise biomarkers for heavy ion radiation exposure, which would enable monitoring of radiation exposure in the context of radiation-related diseases, victims of radiological weapons, the health status of oncology patients, or astronauts. Known methods for measuring radiation dose are based on determining the intensity of ionizing radiation absorption or the radioactive activity of materials (dosimeters). These methods rely on simple probes that detect irradiation, without accounting for the physical properties of chromatin or its accessibility during biomarker identification.
The purpose of the invention was to provide a method for detecting high radiation doses, with potential industrial and medical applications, including radiation diagnostics, monitoring of radiation exposure during radiotherapy, and risk assessment in emergency situations, particularly in scenarios where radiation occurred unexpectedly and no measurement devices had been installed in advance. Notably, all of the aforementioned technical problems have been addressed by the present invention. The essence of the invention is a set of oligonucleotide probes for detecting differential DNA methylation (DMP) for the purpose of predicting the occurrence of radiation-related diseases, wherein the set comprises nine pairs of probes flanking single nucleotides in the genome of lung cells, corresponding to the sequences identified as SEQ ID NO:1-18.

The essence of the invention also relates to a method for *in vitro* prediction of radiation-related disease states by analyzing epigenetic changes in the genome of a subject, the method comprising the steps of:
a) determining DNA methylation and histone protein modification profiles at the specified genomic locations (hg19): chr1:229366001, chr4:11637533, chr6:11233592, chr6:159315559, chr11:67029374, chr12:6339200, chr12:116997095, chr12:116997185, and chr13:111062266, based on a set of predictive probes of SEQ ID NO:1-18 applied to a sample of biological material collected from the subject; and
b) performing a comparative analysis of the DNA methylation levels determined in step a) with those from a healthy individual, and assessing the risk of radiation-related disease states.

In the method according to the invention, the disease state associated with an abnormally altered DNA methylation profile, changed epigenetic regulation of gene expression, and increased patient mortality is lung cancer, selected from non-small cell lung carcinoma or squamous cell lung carcinoma.

Preferably, in the method according to the invention, step a) is performed using techniques involving amplification of DNA fragments (ddPCR, qRT-PCR), sequencing methods based on synthesis, ligation, or the nanopore method, or by DNA restriction analysis using methylation-sensitive restriction endonucleases followed by DNA amplification by PCR.

Preferably, the biological material sample consists of isolated human lung cells.
An advantage of the solution according to the invention is the use of an innovative analysis that combines changes in DNA methylation with the presence of histone modifications characteristic of active chromatin regions. An important aspect of the invention is the identification of regulatory sites located distantly from genes (so-called enhancers) which are situated at a significant distance from the transcription start site and are not associated with classically studied CpG islands. This constitutes a non-standard methodology, as researchers typically focus on methylation changes within CpG islands located in gene promoters (i.e., regulatory regions located approximately 2,000 base pairs upstream of the transcription start site). The inventors have demonstrated that the selected DNA methylation regions are located at the boundary of compact, inactive heterochromatin, which indicates their important role in the long-term regulation of gene expression following irradiation. The integration of information on the three-dimensional spatial organization of chromatin with the analysis of active regulatory elements represents an innovative aspect of the solution.

Another advantage of the invention is the demonstration that specific histone modifications within enhancer regions are critical for the induction of DNA methylation changes in response to irradiation, whereby methylation changes occurring outside CpG islands and promoter regions gain particular significance. In particular, the presence of defined epigenetic marks predisposes a given genomic region to changes following exposure to heavy ions, enabling the narrowing of candidate biomarkers of irradiation. The solution according to the invention is based on the integration of the physical properties of chromatin with DNA methylation analysis, which enables more comprehensive and precise identification of irradiation markers and significantly enhances both diagnostic accuracy and utility. Furthermore, the method according to the invention allows for the detection of irradiation without the need for expensive or specialized equipment, which constitutes a major advantage over existing solutions. Unlike conventional dosimeters, which must be worn during exposure, the method according to the invention permits the assessment of radiation exposure even after a significant delay, providing a unique application in the retrospective analysis of radiation incidents.

The utility of the invention is broad and encompasses both medical and industrial applications. Owing to the absence of a requirement for conventional radiation detectors, the method according to the invention is easier to implement, which is of particular relevance in emergency scenarios. Additionally, it can be applied to estimate the effects of unexpected radiation exposure, such as in the case of equipment malfunction or the deployment of radiological weapons, as well as in the context of risk assessment for long-term space missions and health monitoring of astronauts.

The invention also has potential for use in monitoring radiation exposure in industrial environments, such as nuclear power plants, facilities processing radioactive materials, or in cases of medical equipment failure, where conventional radiation detection methods may be insufficient. By identifying specific epigenetic changes in the genome of an individual resulting from exposure to heavy ion radiation, the method according to the invention enables rapid assessment of the hazard, even in situations where standard dosimetry devices are unable to accurately quantify the radiation dose.

In addition, the method according to the invention is highly sensitive to epigenetic changes that influence cellular processes, allowing for the detection of radiation exposure with high precision, even long after the exposure event. As a result, it can be used in retrospective analyses, such as the assessment of radiation exposure following nuclear accidents, reactor failures, or in evaluating the radiotherapy history of patients, i.e., in cases where conventional detection methods may be ineffective or inapplicable. Another potential area of application is space. In long-duration space missions, where astronauts are continuously exposed to space radiation, monitoring their radiation exposure is essential for protecting their health.

In the medical field, the invention can be used to diagnose radiation-related disease states, such as radiation-induced cancers. Since alterations in DNA methylation are critical to the process of cancer development, the method according to the invention may serve as a diagnostic tool for cancers, particularly those associated with exposure to high doses of radiation, as in the case of individuals undergoing radiotherapy. Analysis of methylation profiles, based on specific genomic alterations, can support the identification of patients at elevated risk who require further monitoring or therapeutic intervention. Specifically, for the observed set of methylation sites, analysis of data from the MethSurv database demonstrated that the presence of these changes is associated with a 17% increased risk of death from non-small cell lung carcinoma, and nearly a 30% increase in the case of squamous cell lung carcinoma. This underscores the potential of the method as a prognostic tool in oncology, capable of supporting clinical decisions related to patient monitoring and personalized treatment strategies.

In summary, the invention provides an innovative approach to detecting exposure to ionizing radiation, based on the precise analysis of epigenetic changes in the genome. Its applicability in medical diagnostics, astronaut health monitoring, the nuclear industry, and emergency response creates new opportunities in the areas of risk assessment, exposure monitoring, and protection against the effects of radiation. By integrating chromatin accessibility data with DNA methylation analysis, the invention enables not only more accurate radiation detection but also retrospective evaluation, which constitutes a significant advantage over traditional radiation detection methods.
The subject matter of the invention is presented with reference to the accompanying figures, in which:
Fig. 1 illustrates a schematic representation of the selection process for a DNA methylation signature;
Fig. 2 illustrates a schematic division of the cell nucleus into spatial layers;
Fig. 3 illustrates a single DNA methylation probe (DMP) and a differentially methylated region (DMR) comprising multiple DNA methylation probes.
The invention is further illustrated by the following embodiments.

### Embodiments

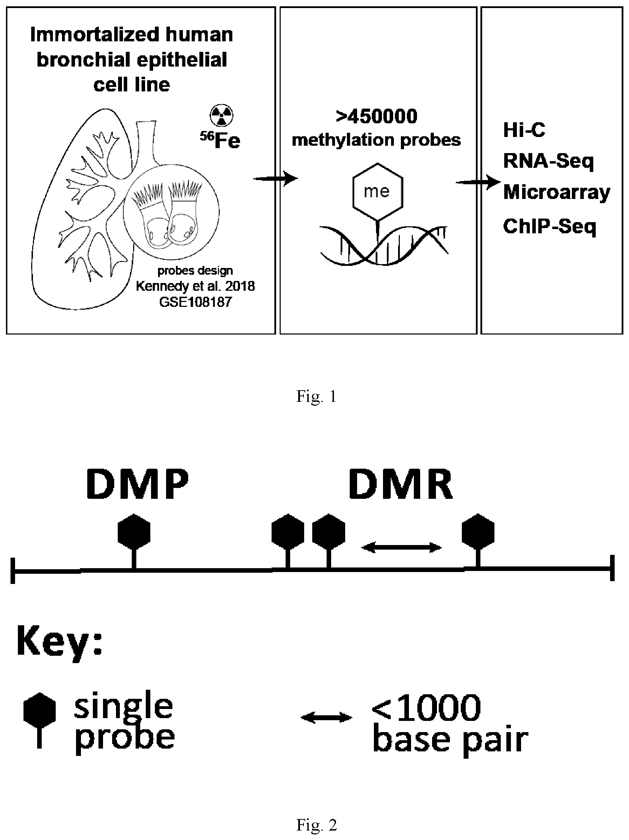

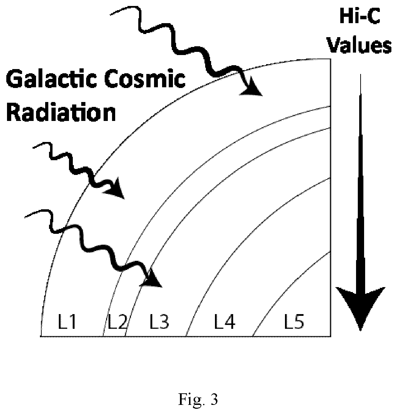

Analyses were conducted using publicly available data (GSE108187; https://www.ncbi.nlm.nih.gov/geo) derived from three independent experimental replicates of the HBEC3-KT lung cell line (Fig. 1), and subsequently validated using eight independent gene expression datasets from irradiated mice and astronauts. Utilizing an integrated multiomics approach, a comprehensive and multi-directional analysis of epigenetic changes was performed, combining data on chromatin accessibility (ChIP-seq), spatial chromatin organization (Hi-C), DNA methylation profiles, and gene expression (RNA-seq). This integrative strategy enabled the identification of specific genomic loci exhibiting qualitative changes in methylation following irradiation, within the context of three-dimensional chromatin architecture and transcriptional activity (Fig. 2). The analyses revealed regions that were initially unmethylated but became methylated after radiation exposure. Subsequently, the three-dimensional localization of these regions within the genome was determined using a method of spatial segmentation of the cell nucleus into concentric layers (Fig. 3).

The dataset comprised 102 immortalized samples of a human bronchial epithelial cell line (HBEC3-KT), obtained by transducing normal human bronchial epithelial cells (HBECs) with mouse Cdk4 and human TERT (hTERT). The cells were cultured in a 5% CO₂ atmosphere at 37°C and passaged twice per week over a period of three months. Three biological replicates were independently irradiated with 56Fe ions at doses of 0, 0.1, 0.3, or 1 Gy (linear energy transfer, LET = 170 keV/µm; beam energy = 600 MeV/u).

To investigate the effect of heavy iron ion irradiation on DNA methylation patterns, the following pairwise comparisons were established: (1) 56Fe at 0.3 Gy versus 28Si at 0.3 Gy; and (2) 56Fe at 1 Gy versus 28Si at 1 Gy. DNA methylation was analyzed using M values (log₂ ratio of methylated probe intensity to unmethylated probe intensity). Differential methylation analysis was performed using 485,061 probes in fixed comparisons, applying the dmpFinder function from the Minfi package. Differentially methylated probes (DMPs) were filtered based on the following criteria: q-value < 0.05; absolute mean difference in M values between compared samples ≥ 0.58; and absolute mean difference in M values relative to unirradiated controls ≥ 0.58. For the purpose of the presented tool, genomic loci (hg19) were selected in which qualitative changes in DNA methylation occurred: chr1:229366001, chr4:11637533, chr6:11233592, chr6:159315559, chr11:67029374, chr12:6339200, chr12:116997095, chr12:116997185, and chr13:111062266. These included sites that were originally methylated and became unmethylated following irradiation, or originally unmethylated sites that became methylated.

To study chromatin conformation in three-dimensional space, Hi-C data for the human lung fibroblast cell line IMR-90 (Series 4DNFIHM89EGL; 4DNFIMNT2VYL, available at https://data.4dnucleome.org/) were used. Using these data, each DMP probe was assigned a Hi-C value corresponding to its spatial orientation within the nucleus. Based on the assigned values, a method was applied to divide the cell nucleus into five concentric layers, designated from the outermost as "L1" to the innermost as "L5", using all probes included in the invention. The boundaries of the nuclear layers were defined using six quantile values (0%, 20%, 40%, 60%, 80%, and 100%), such that each layer contained an equal portion of the genome.

In previous studies, the inventors demonstrated that changes in DNA methylation are most pronounced in regions enriched with histone modifications. Therefore, to identify active genomic regions exhibiting the greatest changes in DNA methylation, probes were selected that overlapped with regions containing acetylation of lysine 27 on histone H3 (H3K27ac). Subsequently, to identify so-called gene transcription enhancers, transcription start sites (TSS) were excluded from these regions. The remaining DMP probes were assigned genomic locations based on their relative position to CpG islands, which are typically located upstream of promoter regions. Probes located at a distance from CpG islands, in regions referred to as "OpenSea," were selected for further analysis.

The designed probes containing the sequences SEQ ID NO:1-18 are artificially synthesized, functionally optimized oligonucleotide sequences that do not occur in nature and have been specifically engineered to detect DNA methylation induced by exposure to heavy ion radiation. Their structure and hybridization conditions have been adapted to allow for highly specific and sensitive detection of methylation changes. As such, the probes according to the invention constitute a technical diagnostic tool with significant clinical potential, extending beyond naturally occurring molecular mechanisms and enabling practical application in monitoring patients after exposure to heavy ion radiation, including within the framework of precision oncology.

The selected genomic signature corresponds to regions where both qualitative and the most significant quantitative changes in DNA methylation occurred following irradiation, and which persist for at least 53 days. Moreover, these regions are spatially localized within the nuclear layers where the radiation beam induced the greatest quantitative changes in DNA methylation. These loci are directly associated with active and accessible chromatin regions marked by the histone modification H3K27ac, yet are located at a distance from transcription start sites and CpG islands that is, in genomic regions situated between compact, inactive heterochromatin domains.

The method for determining the *in vitro* prediction of radiation-related disease states, such as radiation-induced cancers, intended to assess changes in DNA methylation in lung cells, comprises the following steps:
1. Collection of biological material. Lung tissue is collected for analysis in accordance with applicable bioethical standards. It is recommended that samples be immediately frozen in liquid nitrogen and stored at -80 °C until further processing.
2. DNA isolation. Genomic DNA is isolated from tissue using a commercial kit suitable for DNA extraction from tissue, such as the Qiagen DNeasy Blood & Tissue Kit (#69504). Following isolation, DNA concentration and quality are assessed using spectrophotometry. For optimal results, the recommended amount of input DNA ranges from 200 to 500 ng.
3. Bisulfite conversion of DNA. Sodium bisulfite treatment is performed using the Zymo EZ DNA Methylation-Gold Kit (#D5005). During this reaction, unmethylated cytosines are converted to uracils, whereas methylated cytosines remain unchanged, enabling differentiation of methylation status in subsequent analysis.
4. DNA restriction analysis using methylation-sensitive restriction endonucleases. The DNA is subjected to enzymatic digestion with restriction endonucleases that specifically recognize methylated or unmethylated sites.
5. Analysis by droplet digital PCR (ddPCR). Following DNA conversion and restriction digestion, the sample is diluted to a concentration between 1 and 5 ng/µL. A reaction mixture is prepared using the set of oligonucleotide probes according to the invention (serving as ddPCR primers) and the QX200^{™} ddPCR^{™} EvaGreen Supermix reagent (#1864033). The reaction is conducted using the Bio-Rad QX200 ddPCR system. After droplet generation, fluorescence signal amplification and analysis are performed, allowing for precise quantification of DNA copies exhibiting specific methylation patterns.
6. Data analysis. Data interpretation is performed using the Bio-Rad QuantaSoft software, which calculates the number of positive droplets based on fluorescent signal intensity and estimates the DNA methylation level in the regions of interest. In a typical experiment, approximately 15,000 droplets are analyzed, and the signal-to-noise ratio (positive to negative droplet fluorescence) should be at least 3.

### Bibliography

[1] Kennedy, E. M. et al. Galactic cosmic radiation induces persistent epigenome alterations relevant to human lung cancer. Sci. Rep. 8(1), 6709. https://doi.org/10.1038/S41598-018-24755-8 (2018).
[2] Nwanaji-Enwerem, J. C. et al. In vitro relationships of galactic cosmic radiation and epigenetic clocks in human bronchial epithelial cells. Environ. Mol. Mutagen. 63(4), 184-189. https://doi.org/10.1002/em.22483 (2022).
[3] Perdyan, A., Jakalski, M., Horbacz, M. et al. Chromosomal positioning and epigenetic architecture influence DNA methylation patterns triggered by galactic cosmic radiation.Sci Rep 14, 1324 (2024). https://doi.org/10.1038/s41598-024-51756-7
[4] Wang, X., Lee, R., Alver, B. et al. SMARCB1-mediated SWI/SNF complex function is essential for enhancer regulation. Nat Genet 49, 289-295 (2017). https://doi.org/10.1038/ng.3746
[5] Mieczkowski, J., Cook, A., Bowman, S. et al. MNase titration reveals differences between nucleosome occupancy and chromatin accessibility. Nat Commun 7, 11485 (2016). https://doi.org/10.1038/ncomms11485
[6] Mueller B, Mieczkowski J, Kundu S, et al. Widespread changes in nucleosome accessibility without changes in nucleosome occupancy during a rapid transcriptional induction. Genes Dev 31(5):451-462. (2017). doi: 10.1101/gad.293118.116.
[7] Deaton AM, Gómez-Rodriguez M, Mieczkowski J, et al. Enhancer regions show high histone H3.3 turnover that changes during differentiation. Elife, 5:e15316. 2016. doi: 10.7554/eLife.15316.
[8]Stepniak, K., Machnicka, M.A., Mieczkowski, J. et al. Mapping chromatin accessibility and active regulatory elements reveals pathological mechanisms in human gliomas. Nat Commun 12, 3621 (2021). https://doi.org/10.1038/s41467-021-23922-2
[9]Ciechomska, I.A., Gielniewski, B., Wojtas, B. et al. EGFR/FOXO3a/BIM signaling pathway determines chemosensitivity of BMP4-differentiated glioma stem cells to temozolomide.Exp Mol Med 52, 1326-1340 (2020). https://doi.org/10.1038/s12276-020-0479-9
[10]Tiwari V, Prajapati B, Asare Y, et al. Innate immune training restores pro-reparative myeloid functions to promote remyelination in the aged central nervous system. Immunity, 57(9):2173-2190.e8. (2024). doi: 10.1016/j.immuni.2024.07.001.
[11]Leszczynska KB, Freitas-Huhtamäki A, Jayaprakash C, et al. H2A.Z histone variants facilitate HDACi-dependent removal of H3.3K27M mutant protein in pediatric high-grade glioma cells. Cell Rep, 43(2):113707. (2024). doi: 10.1016/j.celrep.2024.113707

## Claims

1. A set of differentially methylated oligonucleotide probes (DMPs) for predicting the occurrence of radiation-related disease states, **characterized in that** the set comprises eighteen probes, having the sequences identified as SEQ ID NO:1-18.

2. A method for *in vitro* prediction of the occurrence of radiation-related disease states by analyzing epigenetic changes in the genome of an individual, **characterized in that** it comprises the steps of:
a) determining DNA methylation profiles and histone protein modifications at the following genomic locations (hg19): chr1:229366001, chr4:11637533, chr6:11233592, chr6:159315559, chr11:67029374, chr12:6339200, chr12:116997095, chr12:116997185, and chr13:111062266, using a set of predictive probes SEQ ID NO:1-18 in a sample of biological material collected from the individual; and
b) performing a comparative analysis of the DNA methylation levels determined in step a) with those of a healthy individual and determining the risk of radiation-related disease states.

3. The method according to claim 2, **characterized in that** the disease state associated with an abnormally altered DNA methylation profile, changed epigenetic regulation of gene expression, and increased patient mortality is lung cancer, selected from non-small cell lung carcinoma or squamous cell lung carcinoma.

4. The method according to claim 2, **characterized in that** step a) is performed using methods involving amplification of DNA fragments (ddPCR, qRT-PCR), sequencing methods based on synthesis, ligation, or nanopore method, or DNA restriction analysis using methylation-sensitive restriction endonucleases, followed by DNA amplification by PCR.

5. The method according to claim 2, **characterized in that** the sample of biological material consists of isolated human lung cells.
